# FASCICULE DE BREVET EUROPEEN

(11) **EP 0 528 706 B1**
(45) Date de publication et mention de la délivrance du brevet: **16.10.1996**
(21) Numéro de dépôt: 92402058.9
(22) Date de dépôt: 16.07.1992
(51) Int. Cl.: A61B 17/58, A61B 17/70

(54) **Implant pour dispositif d'ostéosynthèse notamment du rachis et dispositif correspondant pour sa mise en place**
Implantat für osteosynthetische Vorrichtung, insbesondere für die Wirbelsäule und zugehörende Vorrichtung zu deren Befestigung
Implant for osteosynthesis device, notable for the spine, and associated device for application the same

(30) Priorité: 19.08.1991 FR 9110402
(43) Date de publication de la demande: 24.02.1993
(73) Titulaire: SOCIETE DE FABRICATION DE MATERIEL ORTHOPEDIQUE SOFAMOR, F-75016 Paris (FR)
(72) Inventeur: Cotrel, Yves Paul Charles, F-75016 Paris (FR)
(74) Mandataire: Martin, Jean-Paul

(56) Documents cités:
- EP-A- 0 348 272
- EP-A- 0 441 729
- EP-A- 0 443 892
- US-A- 4 805 602

## Description

La présente invention a pour objet un implant pour dispositif d'ostéosynthèse, notamment du rachis, comprenant une partie destinée à l'ancrage osseux et un corps de fixation sur une tige, dans lequel le corps comprend deux branches latérales délimitant entre elles un canal débouchant sur une partie postérieure du corps et ouvert de part et d'autre du corps pour pouvoir recevoir la tige, cet implant comprenant également un bouchon fileté adapté pour pouvoir être vissé dans des taraudages formés sur les parois intérieures des deux branches latérales. Un tel implant est connu par le document EP-A-348 272.

Le document EP-A-443 892 est cité au titre de l'état de la technique selon l'article 54(3) et (4) CBE pour les Etats désignés suivants : BE, CH, DE, ES, GB, IT, LI, LU, NL et SE. Ce document décrit un implant pour dispositif d'ostéosynthèse, notamment du rachis, comprenant une partie destinée à l'ancrage osseux et un corps de fixation sur une tige, dans lequel le corps comprend deux branches latérales délimitant entre elles un canal débouchant sur une partie postérieure du corps et ouvert de part et d'autre du corps pour pouvoir recevoir la tige, cet implant comprenant également un bouchon fileté adapté pour pouvoir être vissé dans des taraudages formés sur les parois intérieures des deux branches latérales, comprenant entre le bouchon et la tige, un élément de liaison fixé à un moyen de positionnement par rapport aux branches du corps, cet élément étant une lame présentant, du côté de la tige, une surface s'appliquant sur la surface de la tige.

Suivant l'invention, l'implant comprend, entre le bouchon et la tige, un élément fixé à un moyen de positionnement par rapport aux branches du corps, et cet élément est une lame incurvée présentant, du côté de la tige, une surface concave adaptée pour s'appliquer sur la surface de la tige.

Ainsi la surface de la lame en contact avec la tige est très importante, ce qui permet de mieux répartir la pression de contact et donc de l'abaisser, et aussi d'augmenter l'inertie en flexion de la lame par rapport à une lame plate.

Suivant un mode de réalisation, la surface concave de la lame est une portion de cylindre dont le rayon de courbure correspond sensiblement à celui de la surface de la tige, la lame ayant ainsi sensiblement une forme en tuile romaine.

Suivant une autre particularité de l'invention, la lame est coupée sensiblement en son milieu et ainsi constituée de deux demi-lames séparées par une fente sensiblement perpendiculaire à l'axe de la tige.

Ces deux demi-lames permettent un meilleur contact sur une tige courbe ou inclinée dans le canal de la vis.

D'autres particularités et avantages de l'invention apparaitront au cours de la description qui va suivre, faite en référence aux dessins annexés qui en illustrent trois formes de réalisation à titre d'exemples non limitatifs.

La Fig. 1 est une vue en perspective éclatée d'une forme de réalisation de l'implant selon l'invention, à échelle très agrandie.

La Fig. 2 est une vue mi-coupe, mi-élévation suivant 2-2 de la Fig. 1 de l'implant dont les divers éléments ont été assemblés ainsi que d'un outil d'assemblage de ces éléments.

La Fig. 3 est une vue de dessous de la pastille, visible aux Fig. 1 et 2, constituée par la lame et sa bague de fixation par rapport aux branches du corps.

La Fig. 4 est une vue en perspective à échelle agrandie d'une seconde forme de réalisation de la pastille de l'implant selon l'invention, constituée par la lame et son moyen de positionnement.

La Fig. 5 est une vue analogue à la Fig. 4 d'une variante de réalisation de la pastille.

La Fig. 6 est une vue en élévation, à échelle agrandie et dans l'axe de la tige, de l'implant équipé de la pastille de blocage de la Fig. 4.

L'implant 1 représenté aux dessins est destiné à un dispositif d'ostéosynthèse non représenté, notamment du rachis. Il comprend une partie 2, destinée à l'ancrage osseux, ici constituée par une tige filetée, qui peut être remplacée par un crochet.

L'implant comprend également un corps 3 de fixation sur une tige 4 à aspérités, par exemple une tige moletée ou à pointes de diamant. Le corps 3 comprend deux branches latérales 5 débouchant sur une partie postérieure du corps 3 et ouvert de part et d'autre de ce dernier pour pouvoir recevoir la tige 4. Le corps 3 a ainsi une section en U dans un plan transversal au canal 5.

L'implant comprend un bouchon 8 pourvu d'un filetage 9 et dans lequel est usiné un évidement 11 convenablement profilé pour recevoir un outil correspondant 12 de vissage du bouchon 8, dans des taraudages 13 formés sur les parois intérieures des deux branches latérales 5.

L'implant 1 comporte un élément de liaison entre le bouchon 8 et la tige 4, constitué par une lame 14 solidaire d'une bague 15 de positionnement de la lame 14 par rapport aux branches 5, la pastille 16 constituée par la lame 14 et la bague 15 étant réalisée d'une seule pièce par emboutissage d'une tôle, ce qui permet d'importantes économies de fabrication.

La lame 14 s'étend d'une extrémité à l'autre du canal 6, sa largeur étant sensiblement inférieure à la largeur de l'entrée dudit canal.

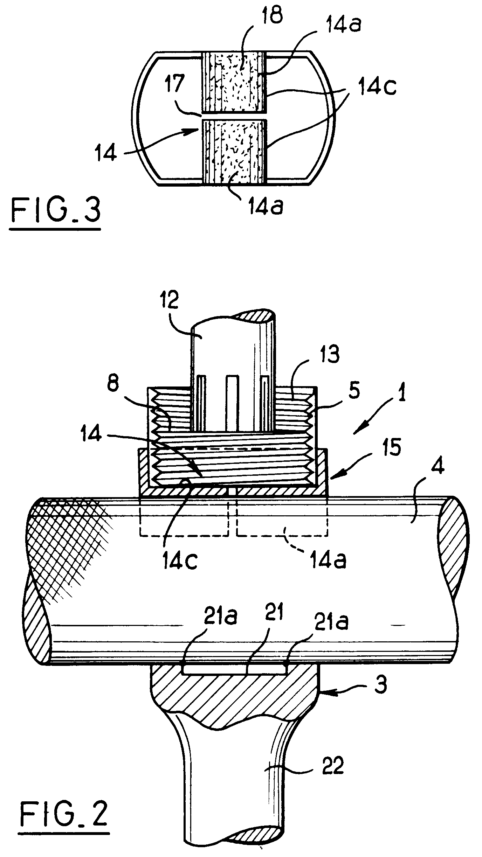

La lame 14 présente, du côté de la tige 4, une surface concave 14a adaptée pour s'appliquer sur la surface de la tige 4, ainsi qu'une surface convexe correspondante 14b du côté du bouchon 8. L'ensemble de la lame 14 forme ainsi une portion de cylindre lui donnant une forme générale de tuile romaine, dont le rayon de courbure correspond sensiblement à celui de la surface de la tige 4. De préférence comme représenté, la lame 14 est coupée sensiblement en son milieu et ainsi constituée de deux demi-lames 14c séparées par une fente 17 sensiblement perpendiculaire à l'axe de la tige 4. Dans les surfaces concaves 14a des demi-lames 14c sont avantageusement ménagées des empreintes 18 (Fig. 3) négatives des aspérités de la tige 4, par exemple des pointes de diamant.

La bague 15 de la pastille 16 est dimensionnée pour entourer les branches 5 lorsqu'elle est mise en place sur le corps 3, par insertion des branches 5 dans les passages réservés entre les bords longitudinaux de la lame 14 et la paroi intérieure de la bague 15. Cette dernière comporte deux portions rectilignes parallèles 15a formant des plats perpendiculaires à l'axe de la tige 4 et solidaires des extrémités opposées des demi-lames 14c, ainsi que deux portions circulaires 15b reliant entre elles les extrémités opposées des plats 15a. Les portions circulaires 15b ont une courbure correspondant à celle de la surface extérieure des branches 5, sur lesquelles elles viennent s'appliquer lorsque la pastille 16 est montée sur le corps 3.

Le canal 6 présente un fond 19 dans la zone centrale duquel est aménagé un évidement 21 s'étendant dans la direction de l'axe de la tige 4. La tige 2 est pourvue d'un filetage 2a dont l'extrémité voisine du corps 3 est séparée de ce dernier par une zone lisse 22, dont la longueur 1, pour une longueur totale de vis égale, est supérieure à la longueur habituelle des zones lisses des vis connues. En effet cette longueur 1 est de préférence au moins sensiblement égale à la longueur des branches 5 du corps 3.

Un bloc 23 de matière souple, choisie de préférence en une matière plastique appropriée, est utilisé avantageusement pour la mise en place des éléments de serrage de la tige 4 dans le corps 3, c'est-à-dire la pastille 16 et le bouchon 8. Ce bloc présente une forme extérieure similaire à celle de la bague 15 et est dimensionné de manière que, après compression, l'une de ses faces puisse venir s'engager à l'intérieur de la bague 15, laquelle est ainsi provisoirement solidarisée avec le bloc 23. Dans ce dernier est agencé un logement 24 de réception du bouchon 8, ce logement 24 débouchant donc sur la face du bloc 23 tournée vers la pastille 16 et la tige 4, lorsque les divers éléments sont positionnés pour leur assemblage. Le logement 24 se prolonge, du côté du bloc 23 opposé à la pastille 16, par un passage central 20 permettant l'introduction de l'extrémité de l'outil 12, convenablement profilé pour venir s'emboîter dans le profil de l'ouverture 11 du bouchon 8 afin de permettre son vissage. Le logement 24 est d'autre part dimensionné pour que le bouchon 8 fasse partiellement saillie du bloc 23 lorsqu'il est en place dans ce logement, afin que cette extrémité saillante puisse venir en prise avec les taraudages 13.

La mise en place par le chirurgien des éléments de serrage 8 et 16 à l'aide du bloc souple 23, s'exécute comme suit :

Tout d'abord le bouchon 8 est introduit dans son logement 24, puis la face du bloc 23 dont fait légèrement saillie le bouchon 8 est comprimée pour pouvoir être introduite à l'intérieur de la bague 15, dont le contour correspond à celui du bloc 23, auquel elle est ainsi provisoirement assujettie par compression élastique du bloc. Au moyen de l'outil 12 engagé dans le passage 20 et le logement 24, le chirurgien approche alors l'ensemble de ces trois pièces 23, 8, 16 du corps 3, sur les branches 5 duquel il fait coulisser la bague 15. La lame 14 vient se positionner dans le canal 6, près de la surface cylindrique de la tige 4.

La manoeuvre de l'outil 12 entraîne la rotation du bouchon 8 et son vissage progressif dans les taraudages 13. Le déplacement du bouchon 8 entraîne la pastille 16, dont les demi-lames 14c et les plats 15a viennent s'appliquer fermement sur la tige 4. A la fin du vissage du bouchon 8, le bloc 23 est complètement désolidarisé de la pastille 16 et du bouchon 8 et peut alors être jeté.

En plus des avantages déjà mentionnés, l'implant selon l'invention présente les suivants :
- L'augmentation de l'inertie en flexion de la lame 14 obtenue par sa forme incurvée complémentaire de la surface de la tige 4, évite une déformation locale de cette lame entre le bouchon 8 et la tige 4. Cette amélioration réduit très sensiblement la pression spécifique nécessaire sur la tige 4 et assure un meilleur centrage de la lame 14 sur la tige, qui a son tour permet un positionnement des empreintes 18 de la lame 14 sur les aspérités (pointes de diamant) de la tige 4. Corrélativement est évitée une tendance au matage des zones en contact entre le bouchon et la lame, ainsi qu'entre cette dernière et la tige. La résistance à la flexion alternée de la tige dans le corps est donc augmentée, de même que la résistance à la fatigue de l'implant.
- Les empreintes négatives 18 améliorent très sensiblement la résistance au glissement de l'assemblage et permettent de réduire la force de serrage nécessaire pour assurer des caractéristiques convenables à l'implant.
- La constitution de la lame 14 en deux demi-lames 14c permet un meilleur contact en deux zones de la lame 14 sur la tige 4, particulièrement avantageux si cette dernière est courbe ou inclinée dans le canal 6, la surface de contact étant ainsi augmentée et donc la résistance à l'arrachement.
- La géométrie de la bague 15 réduit la largeur de l'implant 1 dans la direction de la tige 4, grâce à la réalisation des portions rectilignes 15a perpendiculaires à la tige 4, ce qui favorise les montages courts, notamment dans la zone lombaire du rachis où les courbures sont très importantes et rapprochent les corps des vis.
- Les deux parties rectilignes 15a de la bague 15 sont proches du filetage 9 du bouchon 8. On peut alors, au moyen d'un outil connu dont dispose le chirurgien, déformer les deux plats 15a et serrer le bouchon 8 en déformant ses filets, ce qui assure ainsi un freinage du bouchon 8 écartant tout risque de démontage intempestif de ce bouchon après pose de l'implant dans le patient. La réalisation des portions rectilignes 15a constitue donc un avantage technique important.

La bague 15 assure le maintien des deux branches 5 autour du bouchon 8, dont elles ne peuvent ainsi s'écarter après vissage. Le contour de la bague 15 épouse au mieux celui du corps 3 et assure donc une meilleure tenue à l'arrachement du bouchon 8.

L'évidement 21 ménagé dans le fond 19 du canal 6 assure à la tige 4 un appui en deux points ou deux zones 21a, et de ce fait une meilleure stabilité de la tige 4 en flexion, surtout dans le cas pratiquement général d'une tige courbée.

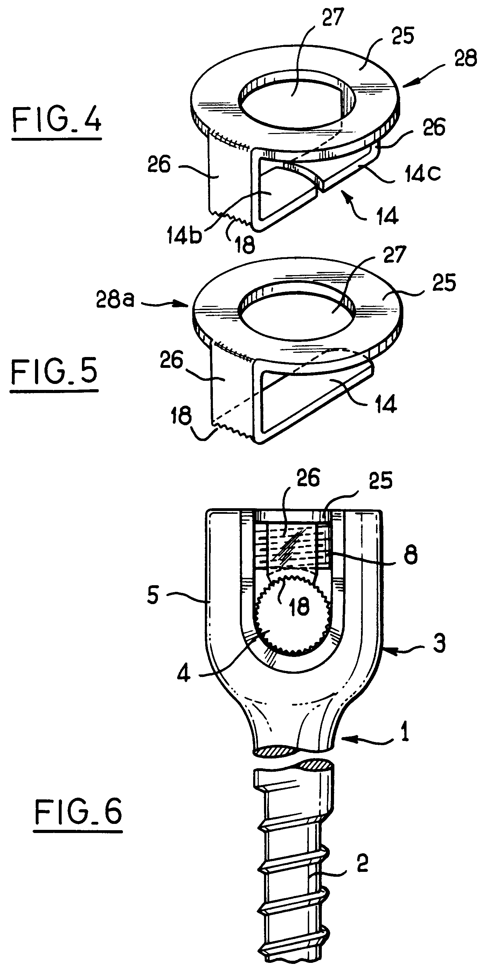

Dans la seconde forme d'exécution de l'invention, illustrée aux Fig. 4 et 6, le moyen de positionnement de la lame 14, scindée en deux demi-lames 14c, séparées par la fente 17, est un anneau 25 relié aux deux demi-lames incurvées 14c par deux joues latérales 26. Ces dernières, diamétralement opposées, forment sensiblement un angle droit avec l'anneau 25 et se prolongent chacune par une demi-lame 14c, délimitant également un angle sensiblement droit avec la joue associée 26.

L'ensemble des éléments 14c, 25 et 26 constitue une pastille 28 réalisée d'une seule pièce à partir d'une lame ou plaque convenablement formée et découpée.

Le diamètre extérieur de l'anneau 25 est égal à celui du bouchon 8, diminué de la hauteur de son filetage. Le diamètre de l'ouverture 27 de l'anneau 25 est au moins égal à celui de l'évidement 11 du bouchon 8.

On introduit le bouchon 8 latéralement entre les joues 26 d'une part et entre la lame 14 et l'anneau 25 d'autre part. Après quoi le chirurgien met en place l'ensemble des pièces 28 et 8 dans le canal 6, puis procède au vissage du bouchon 8 au moyen d'un outil traversant l'ouverture 27 de l'anneau 25.

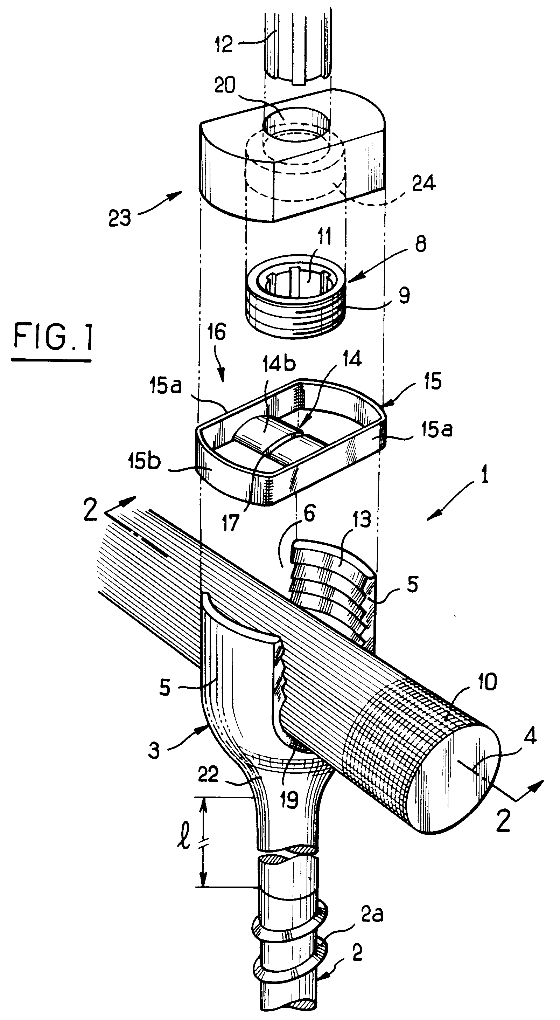

Les avantages de ce mode de réalisation sont les mêmes que ceux des Fig. 1 à 3 (sauf ceux liés à la bague 15) et de plus dans cette seconde réalisation, l'implant présente un encombrement transversal réduit, grâce à la suppression de l'épaisseur de la bague 15 à l'extérieur des branches 5.

D'autre part, les joues 26 peuvent être serrées par étampage contre le bouchon 8 au moyen d'un outil connu approprié, ce qui bloque le bouchon contre tout risque de dévissage spontané après pose de l'implant.

La variante de la pastille 28a représentée à la Fig. 5 diffère de la pastille 28 par le fait qu'elle ne comporte qu'une seule joue latérale 26 et une lame unique 14, qui s'étend dans une direction sensiblement parallèle au plan de l'anneau 25.

Dans les deux réalisations, les pastilles 28 et 28a ont leurs lames 14 incurvées avantageusement creusées d'empreintes 18 comme à la Fig. 3, assurant le blocage de la tige 4 par vissage du bouchon 8.

L'invention n'est pas limitée aux formes de réalisation décrites et peut comporter diverses variantes d'exécution. Ainsi le contour de la bague 15 en deux plats 15a et deux parties circulaires 15b n'est pas obligatoire, de même que la séparation de la lame 14 en deux demi-lames 14c (Fig. 1 à 3). La surface convexe 14b de la lame 14 pourrait être remplacée par une surface plane.

## Revendications (Revendications pour l'(les) Etat(s) contractant(s) suivant(s): GR, MC, DK, PT, AT, FR)

1. Implant (1) pour dispositif d'ostéosynthèse, notamment du rachis, comprenant une partie (2) destinée à l'ancrage osseux et un corps (3) de fixation sur une tige (4), dans lequel le corps comprend deux branches latérales (5) délimitant entre elles un canal (6) débouchant sur une partie postérieure du corps et ouvert de part et d'autre du corps pour pouvoir recevoir la tige, cet implant comprenant également un bouchon fileté (8) adapté pour pouvoir être vissé dans des taraudages (13) formés sur les parois intérieures des deux branches latérales, caractérisé en ce qu'il comprend entre le bouchon et la tige, un élément (14) de liaison fixé à un moyen (15) de positionnement par rapport aux branches du corps, et cet élément est une lame incurvée (14) présentant, du côté de la tige, une surface concave (14a) adaptée pour s'appliquer sur la surface de la tige.

2. Implant selon la revendication 1, caractérisé en ce que la surface concave (14a) de la lame (14) est une portion de cylindre dont le rayon de courbure correspond sensiblement à celui de la surface de la tige (4).

3. Implant selon l'une des revendications 1 et 2, caractérisé en ce que la lame (14) est coupée sensiblement en son milieu et ainsi constituée de deux demi-lames (14c) séparées par une fente (17) sensiblement perpendiculaire à l'axe de la tige (4).

4. Implant selon l'une des revendications 1 à 3, destiné à être associé à une tige (4) dont la surface est revêtue d'aspérités telles que des pointes, caractérisé en ce que dans la surface concave (14a) de la lame (14) sont ménagées des empreintes (18) négatives des aspérités (10) de la tige.

5. Implant selon l'une des revendications 1 à 4, caractérisé en ce que ledit moyen de positionnément est une bague (15) fixée à la lame (14) et qui comporte deux portions rectilignes parallèles (15a) formant des plats perpendiculaires à l'axe de la tige (4), et deux portions circulaires (15b) reliant entre elles les extrémités desdits plats, ces portions circulaires ayant une courbure correspondant à celle de la surface extérieure des branches (5) du corps (3) sur lesquelles elles s'appliquent.

6. Implant selon l'une des revendications 1 à 5, caractérisé en ce que dans la zone centrale du fond (19) du canal (6) délimité par les branches (5) du corps (3), est ménagé un évidement (21) s'étendant dans la direction de l'axe de la tige (4).

7. Implant selon l'une des revendications 1 à 6, caractérisé en ce que la surface (14b) de la lame (14) tournée vers le bouchon est convexe.

8. Implant selon l'une quelconque des revendications 1 à 7, dans lequel la partie destinée à l'ancrage osseux est une tige filetée (2) dont le filetage (2a) est séparé du corps (3) par une zone lisse (22), caractérisé en ce que la zone lisse a une longueur (1) au moins sensiblement égale à la longueur des branches (5) du corps (3).

9. Implant selon l'une des revendications 1 à 4, caractérisé en ce que ledit moyen de positionnement de la lame (14) est un anneau (25) relié à ladite lame par au moins une joue latérale (26) et de préférence deux, l'anneau étant réalisé pour pouvoir s'appliquer entre les branches (5) du corps (3), sur l'extrémité du bouchon (8) opposée à la tige (4).

10. Implant selon la revendication 9, caractérisé en ce qu'il comporte, soit une paire de joues latérales (26) prolongées chacune par une demi-lame (14c), une fente (17) sensiblement perpendiculaire à la tige (4) séparant les deux demi, lames, soit une seule joue latérale (26) prolongée par une lame unique (14), la pastille (28, 28a) constituée par l'anneau, la ou les joue(s) et la lame étant réalisée d'une seule pièce à partir d'une plaque convenablement formée et découpée.

11. Dispositif d'implant d'ostéosynthèse, comportant un implant (1) selon l'une quelconque des revendications 1 à 10, caractérisé en ce qu'il comporte un bloc (23) de matière souple dans lequel est agencé un logement (24) de réception du bouchon fileté (8), dimensionné de manière que ce dernier fasse partiellement saillie du bloc lorsqu'il est en place dans ce logement, et ce bloc est également agencé pour pouvoir s'engager, après compression, à l'intérieur de la bague (15), laquelle peut ainsi être assujettie au bloc du côté du logement (24) du bouchon fileté, un passage central (20) pour un outil (12) de vissage du bouchon (8) étant réalisé dans le bloc souple.

## Revendications (Revendications pour l'(les) Etat(s) contractant(s) suivant(s): BE, CH, DE, ES, GB, IT, LI, LU, NL, SE)

1. Implant (1) pour dispositif d'ostéosynthèse, notamment du rachis, comprenant une partie (2) destinée à l'ancrage osseux et un corps (3) de fixation sur une tige (4), dans lequel le corps comprend deux branches latérales (5) délimitant entre elles un canal (6) débouchant sur une partie postérieure du corps et ouvert de part et d'autre du corps pour pouvoir recevoir la tige, cet implant comprenant également un bouchon fileté (8) adapté pour pouvoir être vissé dans des taraudages (13) formés sur les parois intérieures des deux branches latérales, et ledit implant comprend, entre le bouchon et la tige, un élément (14) de liaison fixé à un moyen (15) de positionnement par rapport aux branches du corps, cet élément étant une lame incurvée (14) présentant, du côté de la tige, une surface concave (14a) adaptée pour s'appliquer sur la surface de la tige.

2. Implant selon la revendication 1, dans lequel la surface concave (14a) de la lame (14) est une portion de cylindre dont le rayon de courbure correspond sensiblement à celui de la surface de la tige (4).

3. Implant selon l'une des revendications 1 et 2, dans lequel la lame (14) est coupée sensiblement en son milieu et ainsi constituée de deux demi-lames (14c) séparées par une fente (17) sensiblement perpendiculaire à l'axe de la tige (4).

4. Implant selon l'une des revendications 1 à 3, destiné à être associé à une tige (4) dont la surface est revêtue d'aspérités telles que des pointes, dans lequel dans la surface concave (14a) de la lame (14) sont ménagées des empreintes (18) négatives des aspérités (10) de la tige.

5. Implant selon l'une des revendications 1 à 4, dans lequel ledit moyen de positionnement est une bague (15) fixée à la lame (14) et qui comporte deux portions rectilignes parallèles (15a) formant des plats perpendiculaires à l'axe de la tige (4), et deux portions circulaires (15b) reliant entre elles les extrémités desdits plats, ces portions circulaires ayant une courbure correspondant à celle de la surface extérieure des branches (5) du corps (3) sur lesquelles elles s'appliquent.

6. Implant selon l'une des revendications 1 à 5, dans lequel dans la zone centrale du fond (19) du canal (6) délimité par les branches (5) du corps (3), est ménagé un évidement (21) s'étendant dans la direction de l'axe de la tige (4).

7. Implant selon l'une des revendications 1 à 6, dans lequel la surface (14b) de la lame (14) tournée vers le bouchon est convexe.

8. Implant selon l'une quelconque des revendications 1 à 7, dans lequel la partie destinée à l'ancrage osseux est une tige filetée (2) dont le filetage (2a) est séparé du corps (3) par une zone lisse (22), dans lequel la zone lisse a une longueur (1) au moins sensiblement égale à la longueur des branches (5) du corps (3).

9. Implant selon l'une des revendications 1 à 4, ledit moyen de positionnement de la lame (14) est un anneau (25) relié à ladite lame par au moins une joue latérale (26) et de préférence deux, l'anneau étant réalisé pour pouvoir s'appliquer entre les branches (5) du corps (3), sur l'extrémité du bouchon (8) opposée à la tige (4).

10. Implant selon la revendication 9, comportant, soit une paire de joues latérales (26) prolongées chacune par une demi-lame (14c), une fente (17) sensiblement perpendiculaire à la tige (4) séparant les deux demi-lames, soit une seule joue latérale (26) prolongée par une lame unique (14), la pastille (28, 28a) constituée par l'anneau, la ou les joue(s) et la lame étant réalisée d'une seule pièce à partir d'une plaque convenablement formée et découpée.

11. Dispositif d'implant d'ostéosynthèse, comportant un implant (1) selon l'une quelconque des revendications 1 à 10, comportant un bloc (23) de matière souple dans lequel est agencé un logement (24) de réception du bouchon fileté (8), dimensionné de manière que ce dernier fasse partiellement saillie du bloc lorsqu'il est en place dans ce logement, et ce bloc est également agencé pour pouvoir s'engager, après compression, à l'intérieur de la bague (15), laquelle peut ainsi être assujettie au bloc du côté du logement (24) du bouchon fileté, un passage central (20) pour un outil (12) de vissage du bouchon (8) étant réalisé dans le bloc souple.

## Claims (Claims for the following Contracting State(s): GR, MC, DK, PT, AT, FR)

1. An implant (1) for an osteosynthesis device, in particular for the spine, comprising a part (2) for the bone anchorage and a body (3) for fixing to a rod (4), in which implant the body comprises two lateral legs (5) bounding therebetween a duct (6) opening into a rear part of the body and open on both sides of the body to receive the rod, this implant also comprising a screwed plug (8) adapted so that it can be screwed into the internal threads (13) formed on the internal walls of the two lateral legs, characterised in that the said implant comprises, between the plug and the rod, a linking member (14) fixed to means (15) for positioning in relation to the legs of the body, and this member is a curved strip (14) having, on the side of the rod, a concave surface (14a) adapted to fit over the surface of the rod.

2. An implant according to Claim 1, characterised in that the concave surface (14a) of the strip (14) is a cylinder portion, the curve radius thereof corresponding substantially to the curve radius of the surface of the rod (4).

3. An implant according to either of Claims 1 or 2, characterised in that the strip (14) is cut substantially in the centre thereof and thus comprises two half-strips (14c) separated by a slit (17) substantially perpendicular to the axis of the rod (4).

4. An implant according to any one of Claims 1 to 3, for being associated with a rod (4) with a surface covered with bumps such as peaks, characterised in that negative impressions (18) of the bumps (10) of the rod are formed in the concave surface (14a) of the strip (14).

5. An implant according to any one of Claims 1 to 4, characterised in that the said positioning means is a ring (15) secured to the strip (14) and which comprises two parallel rectilinear portions (15a) forming flats perpendicular to the axis of the rod (4), and two circular portions (15b) linking therebetween the ends of the said flats, these circular portions having a curve corresponding to the curve of the outer surface of the legs (5) of the body (3) over which they fit.

6. An implant according to any one of Claims 1 to 5, characterised in that a recess (21) extending in the direction of the axis of the rod (4) is formed in the central area of the bottom (19) of the duct (6) bounded by the legs (5) of the body (3).

7. An implant according to any one of Claims 1 to 6, characterised in that the surface (14b) of the strip (14) facing the plug is convex.

8. An implant according to any one of Claims 1 to 7, in which the part for the bone anchorage is a threaded rod (2), the thread (2a) thereof being separated from the body (3) by a smooth area (22), characterised in that the length (1) of the smooth area is at least substantially the same as the length of the legs (5) of the body (3).

9. An implant according to any one of Claims 1 to 4, characterised in that the said means for positioning the strip (14) is a collar (25) linked to the said strip by at least one lateral lug (26), and preferably two, the collar being constructed to be able to fit between the legs (5) of the body (3), over the end of the plug (8) opposite the rod (4).

10. An implant according to Claim 9, characterised in that it comprises either a pair of lateral lugs (26) each extended by a half-strip (14c), a slit (17) substantially perpendicular to the rod (4) separating the two half-strips, or a single lateral lug (26) extended by just one strip (14), the disc-shaped member (28, 28a) constructed using the collar, the lug(s) and the strip and being produced using a single piece from a plate formed and cut in a suitable manner.

11. An osteosynthesis implant device, characterised in that it comprises an implant (1) according to any one of Claims 1 to 10, comprising a block (23) of flexible material in which a housing (24) is arranged to receive the screwed plug (8), and dimensioned such that the said plug projects partially from the block when it is in place in this housing, and this block is also arranged to engage, after compression, inside the ring (15), which can thus be secured to the block from the side of the housing (24) of the screwed plug, a central passage (20) for a tool (12) for screwing the plug (8) being constructed in the flexible block.

## Claims (Claims for the following Contracting State(s): BE, CH, DE, ES, GB, IT, LI, LU, NL, SE)

1. An implant (1) for an osteosynthesis device, in particular for the spine, comprising a part (2) for the bone anchorage and a body (3) for fixing to a rod (4), in which implant the body comprises two lateral legs (5) bounding therebetween a duct (6) opening into a rear part of the body and open on both sides of the body to receive the rod, this implant also comprising a screwed plug (8) adapted so that it can be screwed into the internal threads (13) formed on the internal walls of the two lateral legs, and the said implant comprises, between the plug and the rod, a linking member (14) fixed to means (15) for positioning in relation to the legs of the body, this member being a curved strip (14) having, on the side of the rod, a concave surface (14a) adapted to fit over the surface of the rod.

2. An implant according to Claim 1, in which the concave surface (14a) of the strip (14) is a cylinder portion, the curve radius thereof corresponding substantially to the curve radius of the surface of the rod (4).

3. An implant according to either of Claims 1 or 2, in which the strip (14) is cut substantially in the centre thereof and thus comprises two half-strips (14c) separated by a slit (17) substantially perpendicular to the axis of the rod (4).

4. An implant according to any one of Claims 1 to 3, for being associated with a rod (4) with a surface covered with bumps such as peaks, in which implant negative impressions (18) of the bumps (10) of the rod are formed in the concave surface (14a) of the strip (14).

5. An implant according to any one of Claims 1 to 4, in which the said positioning means is a ring (15) secured to the strip (14) and which comprises two parallel rectilinear portions (15a) forming flats perpendicular to the axis of the rod (4), and two circular portions (15b) linking therebetween the ends of the said flats, these circular portions having a curve corresponding to the curve of the outer surface of the legs (5) of the body (3) over which they fit.

6. An implant according to any one of Claims 1 to 5, in which a recess (21) extending in the direction of the axis of the rod (4) is formed in the central area of the bottom (19) of the duct (6) bounded by the legs (5) of the body (3).

7. An implant according to any one of Claims 1 to 6, in which the surface (14b) of the strip (14) facing the plug is convex.

8. An implant according to any one of Claims 1 to 7, in which the part for the bone anchorage is a threaded rod (2), the thread (2a) thereof being separated from the body (3) by a smooth area (22), in which implant the length (1) of the smooth area is at least substantially the same as the length of the legs (5) of the body (3).

9. An implant according to any one of Claims 1 to 4, [in which] the said means for positioning the strip (14) is a collar (25) linked to the said strip by at least one lateral lug (26), and preferably two, the collar being constructed to be able to fit between the legs (5) of the body (3), over the end of the plug (8) opposite the rod (4).

10. An implant according to Claim 9, comprising either a pair of lateral lugs (26) each extended by a half-strip (14c), a slit (17) substantially perpendicular to the rod (4) separating the two half-strips, or a single lateral lug (26) extended by just one strip (14), the disc-shaped member (28, 28a) constructed using the collar, the lug(s) and the strip and being produced using a single piece from a plate formed and cut in a suitable manner.

11. An osteosynthesis implant device, comprising an implant (1) according to any one of Claims 1 to 10, comprising a block (23) of flexible material in which a housing (24) is arranged to receive the screwed plug (8), and dimensioned such that the said plug projects partially from the block when it is in place in this housing, and this block is also arranged to engage, after compression, inside the ring (15), which can thus be secured to the block from the side of the housing (24) of the screwed plug, a central passage (20) for a tool (12) for screwing the plug (8) being constructed in the flexible block.

## Patentansprüche (Patentansprüche für folgende(n) Vertragsstaat(en): GR, MC, DK, PT, AT, FR)

1. Implantat (1) für eine Osteosynthese-Vorrichtung, insbesondere für die Wirbelsäule, bestehend aus einem Teil (2), der für die Knochenverankerung bestimmt ist und einem Körper (3) zur Befestigung an einer Stange (4), wobei der Körper zwei seitliche Zweige (5) umfaßt, die zwischen sich einen Kanal (6) begrenzen, der in einen hinteren Teil des Körpers mündet und auf beiden Seiten des Körpers offen ist, um die Stange aufnehmen zu können, wobei dieses Implantat auch eine Verschlußschraube (8) umfaßt, die derart angepaßt ist, daß sie in Innengewinde (13) geschraubt werden kann, die an den Innenwandungen der beiden seitlichen Zweige ausgebildet sind dadurch gekennzeichnet, daß dieses Implantat zwischen der Schraube und der Stange ein Verbindungselement (14) umfaßt, das an einem Mittel (15) zur Positionierung in bezug auf die Zweige des Körpers befestigt ist, wobei dieses Element ein gekrümmtes Plättchen (14) ist, das auf Stangenseite eine konkave Fläche (14a) aufweist, die derart ausgeführt ist, daß sie an der Fläche der Stange anliegen kann.

2. Implantat nach Anspruch 1, dadurch gekennzeichnet, daß die konkave Fläche (14a) des Plättchens (14) ein Teil eines Zylinders ist, dessen Krümmungsradius im wesentlichen jenem der Fläche der Stange (4) entspricht.

3. Implantat nach einem der Ansprüche 1 und 2, dadurch gekennzeichnet, daß das Plättchen (14) im wesentlichen in seiner Mitte durchschnitten ist und somit von zwei halben Plättchen (14c) gebildet wird, die durch einen Spalt (17), der im wesentlichen auf die Achse der Stange (4) senkrecht steht, getrennt sind.

4. Implantat nach einem der Ansprüche 1 bis 3, das dazu bestimmt ist, an einer Stange (4) befestigt zu werden, deren Oberfläche mit Unebenheiten, wie beispielsweise Spitzen, überzogen ist, dadurch gekennzeichnet, daß in der konkaven Fläche (14a) des Plättchens (14) negative Eindrücke (18) der Unebenheiten (10) der Stange ausgespart sind.

5. Implantat nach einem der Ansprüche 1 bis 4, dadurch gekennzeichnet, daß das Mittel zur Positionierung ein Ring (15) ist, der an dem Plättchen (14) befestigt ist und zwei geradlinige parallele Teile (15a), die Flachseiten bilden, die auf die Achse der Stange (4) senkrecht stehen, und zwei kreisförmige Teile (15b) umfaßt, die zwischen sich die Enden dieser Flachseiten verbinden, wobei diese kreisförmigen Teile eine Krümmung aufweisen, die jener der Außenfläche der Zweige (5) des Körpers (3), an die sie sich anlegen, entspricht.

6. Implantat nach einem der Ansprüche 1 bis 5, dadurch gekennzeichnet, daß in dem Mittelbereich des Bodens (19) des Kanals (6), der von den Zweigen (5) des Körpers (3) begrenzt wird, eine Aussparung (21) ausgespart ist, die sich in die Richtung der Achse der Stange (4) erstreckt.

7. Implantat nach einem der Ansprüche 1 bis 6, dadurch gekennzeichnet, daß die zu der Schraube gerichtete Fläche (14b) des Plättchens (14) konvex ist.

8. Implantat nach einem der Ansprüche 1 bis 7, bei dem der Teil, der für die Knochenverankerung vorgesehen ist, eine Gewindestange (2) ist, deren Gewinde (2a) von dem Körper (3) durch einen glatten Bereich (22) getrennt ist, dadurch gekennzeichnet, daß der glatte Bereich eine Länge (1) aufweist, die im wesentlichen gleich der Länge der Zweige (5) des Körpers (3) ist.

9. Implantat nach einem der Ansprüche 1 bis 4, dadurch gekennzeichnet, daß das Mittel zur Positionierung des Plättchens (14) ein Ring (25) ist, der an dem Plättchen durch zumindest eine Seitenbacke (26) und vorzugsweise zwei befestigt ist, wobei der Ring derart ausgeführt ist, daß er sich zwischen den Zweigen (5) des Körpers (3) auf dem der Stange (4) gegenüberliegenden Ende der Schraube (8) anlegen kann.

10. Implantat nach Anspruch 9, dadurch gekennzeichnet, daß es entweder ein Paar von Seitenbacken (26), die jeweils durch ein halbes Plättchen (14c) verlängert sind, umfaßt, wobei ein im wesentlichen auf die Stange (4) senkrechter Spalt (17) die beiden halben Plättchen trennt, oder nur eine Seitenbacke (26) umfaßt, die durch ein einzelnes Plättchen (14) verlängert ist, wobei die Tablette (28, 28a), die von dem Ring, der oder den Seitenbacke(n) und dem Plättchen gebildet wird, aus einem Stück aus einer entsprechend geformten und geschnittenen Platte hergestellt ist.

11. Osteosynthese-Implantat-Vorrichtung, bestehend aus einem Implantat (1) nach einem der Ansprüche 1 bis 10, dadurch gekennzeichnet, daß es aus einem Block (23) aus elastischem Material besteht, in dem eine Lagerung (24) für die Aufnahme der Verschlußschraube (8) angeordnet ist, der derart dimensioniert ist, daß letztgenannte teilweise aus dem Block vorspringt, wenn sie sich in dieser Lagerung befindet, und wobei dieser Block auch derart angeordnet ist, daß er nach Zusammendrücken im Inneren des Ringes (15) eingreifen kann, welcher somit mit dem Block auf der Seite der Lagerung (24) der Verschlußschraube verbunden werden kann, wobei ein Mitteldurchgang (20) für ein Werkzeug (12) zum Verschrauben der Schraube (8) in dem elastischen Block vorgesehen ist.

## Patentansprüche (Patentansprüche für folgende(n) Vertragsstaat(en): BE, CH, DE, ES, GB, IT, LI, LU, NL, SE)

1. Implantat (1) für eine Osteosynthese-Vorrichtung, insbesondere für die Wirbelsäule, bestehend aus einem Teil (2), der für die Knochenverankerung bestimmt ist und einem Körper (3) zur Befestigung an einer Stange (4), wobei der Körper zwei seitliche Zweige (5) umfaßt, die zwischen sich einen Kanal (6) begrenzen, der in einem hinteren Teil des Körpers mündet und auf beiden Seiten des Körpers offen ist, um die Stange aufnehmen zu können, wobei dieses Implantat auch eine Verschlußschraube (8) umfaßt, die derart angepaßt ist, daß sie in Innengewinde (13) geschraubt werden kann, die an den Innenwandungen der beiden seitlichen Zweige ausgebildet sind, und wobei dieses Implantat zwischen der Schraube und der Stange ein Verbindungselement (14) umfaßt, das an einem Mittel (15) zur Positionierung in bezug auf die Zweige des Körpers befestigt ist, wobei dieses Element ein gekrümmtes Plättchen (14) ist, das auf Stangenseite eine konkave Fläche (14a) aufweist, die derart ausgeführt ist, daß sie an der Fläche der Stange anliegen kann.

2. Implantat nach Anspruch 1, bei dem die konkave Fläche (14a) des Plättchens (14) ein Teil eines Zylinders ist, dessen Krümmungsradius im wesentlichen jenem der Fläche der Stange (4) entspricht.

3. Implantat nach einem der Ansprüche 1 und 2, bei dem das Plättchen (14) im wesentlichen in seiner Mitte durchschnitten ist und somit von zwei halben Plättchen (14c) gebildet wird, die durch einen Spalt (17), der im wesentlichen auf die Achse der Stange (4) senkrecht steht, getrennt sind.

4. Implantat nach einem der Ansprüche 1 bis 3, das dazu bestimmt ist, an einer Stange (4) befestigt zu werden, deren Oberfläche mit Unebenheiten, wie beispielsweise Spitzen, überzogen ist, wobei in der konkaven Fläche (14a) des Plättchens (14) negative Eindrücke (18) der Unebenheiten (10) der Stange ausgespart sind.

5. Implantat nach einem der Ansprüche 1 bis 4, bei dem das Mittel zur Positionierung ein Ring (15) ist, der an dem Plättchen (14) befestigt ist und zwei geradlinige parallele Teile (15a), die Flachseiten bilden, die auf die Achse der Stange (4) senkrecht stehen, und zwei kreisförmige Teile (15b) umfaßt, die zwischen sich die Enden dieser Flachseiten verbinden, wobei diese kreisförmigen Teile eine Krümmung aufweisen, die jener der Außenfläche der Zweige (5) des Körpers (3), an die sie sich anlegen, entspricht.

6. Implantat nach einem der Ansprüche 1 bis 5, bei dem in dem Mittelbereich des Bodens (19) des Kanals (6), der von den Zweigen (5) des Körpers (3) begrenzt wird, eine Aussparung (21) ausgespart ist, die sich in die Richtung der Achse der Stange (4) erstreckt.

7. Implantat nach einem der Ansprüche 1 bis 6, bei dem die zu der Schraube gerichtete Fläche (14b) des Plättchens (14) konvex ist.

8. Implantat nach einem der Ansprüche 1 bis 7, bei dem der Teil, der für die Knochenverankerung vorgesehen ist, eine Gewindestange (2) ist, deren Gewinde (2a) von dem Körper (3) durch einen glatten Bereich (22) getrennt ist, wobei der glatte Bereich eine Länge (1) aufweist, die im wesentlichen gleich der Länge der Zweige (5) des Körpers (3) ist.

9. Implantat nach einem der Ansprüche 1 bis 4, bei dem das Mittel zur Positionierung des Plättchens (14) ein Ring (25) ist, der an dem Plättchen durch zumindest eine Seitenbacke (26) und vorzugsweise zwei befestigt ist, wobei der Ring derart ausgeführt ist, daß er sich zwischen den Zweigen (5) des Körpers (3) auf dem der Stange (4) gegenüberliegenden Ende der Schraube (8) anlegen kann.

10. Implantat nach Anspruch 9, das entweder ein Paar von Seitenbacken (26), die jeweils durch ein halbes Plättchen (14c) verlängert sind, umfaßt, wobei ein im wesentlichen auf die Stange (4) senkrechter Spalt (17) die beiden halben Plättchen trennt, oder nur eine Seitenbacke (26) umfaßt, die durch ein einzelnes Plättchen (14) verlängert ist, wobei die Tablette (28, 28a), die von dem Ring, der oder den Seitenbacke(n) und dem Plättchen gebildet wird, aus einem Stück aus einer entsprechend geformten und geschnittenen Platte hergestellt ist.

11. Osteosynthese-Implantat-Vorrichtung, bestehend aus einem Implantat (1) nach einem der Ansprüche 1 bis 10, bestehend aus einem Block (23) aus elastischem Material, in dem eine Lagerung (24) für die Aufnahme der Verschlußschraube (8) angeordnet ist, der derart dimensioniert ist, daß letztgenannte teilweise aus dem Block vorspringt, wenn sie sich in dieser Lagerung befindet, und wobei dieser Block auch derart angeordnet ist, daß er nach Zusammendrücken im Inneren des Ringes (15) eingreifen kann, welcher somit mit dem Block auf der Seite der Lagerung (24) der Verschlußschraube verbunden werden kann, wobei ein Mitteldurchgang (20) für ein Werkzeug (12) zum Verschrauben der Schraube (8) in dem elastischen Block vorgesehen ist.
